# EUROPÄISCHE PATENTSCHRIFT

(11) **EP 0 313 800 B1**
(45) Veröffentlichungstag und Bekanntmachung des Hinweises auf die Patenterteilung: **29.06.1994**
(21) Anmeldenummer: 88115318.3
(22) Anmeldetag: 19.09.1988
(51) Int. Cl.: A61F 13/15

(54) **Hygienischer Zellstoffartikel als Einmalartikel und Verfahren zu seiner Herstellung**
Disposable absorbent article and its process of production
Article d'hygiène à jeter et procédé de fabrication

(30) Priorität: 27.10.1987 DE 3736275
(43) Veröffentlichungstag der Anmeldung: 03.05.1989
(73) Patentinhaber: Paul Hartmann Aktiengesellschaft, D-89522 Heidenheim (DE)
(72) Erfinder: Linnebur, Clemens, Dipl.-Ing., D-7920 Heidenheim (DE); Malowaniec, Krzystof, Dipl.-Ing., D-7920 Heidenheim (DE)
(74) Vertreter: Becker, Maria, Dipl.-Phys.

(56) Entgegenhaltungen:
- EP-A- 0 157 649
- US-A- 4 212 302
- US-A- 4 551 191
- US-A- 4 643 726
- US-A- 4 699 808

## Beschreibung

Die Erfindung betrifft einen hygienischen Zellstoffartikel nach dem Oberbegriff des Anspruchs 1.

Aus der EF-A-0 157 649 ist eine Windel mit einem Saugkörper bekannt, der im Mittelbereich eine Verdichtung des Fasermaterials aufweist, um in diesem Bereich eine höhere Kapillarität zu erzielen. Zum Ausgleich des durch die Verdichtung bewirkten Verlustes des Flüssigkeitsaufnahmevermögens sind zusätzlich Hydrogele eingelagert, die im Verstärkungsbereich und gegebenenfalls auch über den gesamten Saugkörper vorzugsweise gleichmässig verteilt eingelagert sind. Infolge der Mittenverstärkung wird hierdurch zwar eine gewisse Mengenkonzentration im Schrittbereich bewirkt, jedoch ergibt sich keine optimale Anpassung dieser superabsorbierenden Stoffe an die tatsächliche Verteilung der anfallenden Flüssigkeit. Auch hat eine solche gleichmässige Verteilung der Quellsubstanzen im Saugkörper den Nachteil, dass im Endbereich der Windel noch hohe Mengen von Quellstoffen vorhanden sind, die in diesem Bereich nicht zur Wirkung kommen, andererseits bei der Fertigung sich dahingehend nachteilig auswirken, dass sie die Schneidmesser abstumpfen. Auch können im Endbereich der Windel die aus Granulaten bestehenden Quellsubstanzen aus der Windel austreten.

Als Quellsubstanzen eignen sich insbesondere Hydrogele bildende Polymere, wie sie beispielsweise in der EP-A 0 205 674 beschrieben sind. Es handelt sich hierbei um hochquellfähige Polymere, die bei Zutritt von Flüssigkeit langsam quellen und sie in einer grossen Menge speichern.

Der Erfindung liegt die Aufgabe zugrunde, den Zellstoffartikel der gattungsgemässen Art so weiterzuentwickeln, dass bei wirtschaftlichem Einsatz der Saugmaterialien eine optimale Flüssigkeitsaufnahmecharakteristik erzielt wird.

Diese Aufgabe wird erfindungsgemäss dadurch gelöst, dass die Quellstoffsubstanzen in Richtung der Windellängsachse vom Verstärkungsbereich nach aussen abnehmend entsprechend dem Verteilungsprofil der anfallenden Flüssigkeit derart aufgetragen sind, dass die Konzentration der Substanzen im Saugkörper im Verstärkungsbereich 8 % bis 40 % und in den Taillenbereichen 1 % bis 7 % des Gewichtes des Saugkörpers beträgt. Hierdurch ist auch gewährleistet, dass in den Endbereichen der Windel nur wenig Quellsubstanzen vorhanden sind, so dass beim Zerschneiden der Produktbahn in Einzelteile die Schneidmesser geschont werden.

Bei einer bevorzugten Ausführungsform beträgt die Konzentration der Quellstoffsubstanzen im Schrittbereich des Saugkörpers ca. 10% bis 15% und in den Taillenbereichen 2% bis 5%.

Ein erfindungsgemässes Verfahren zur Herstellung eines solchen Hygieneartikels ist Gegenstand der Unteransprüche 4 bis 7.

In der nachfolgenden Beschreibung wird die Erfindung anhand von Zeichnungen näher erläutert.

In der Zeichnung zeigen:
- Fig. 1: eine Draufsicht auf einen Einmalartikel in Form einer ausgebreiteten Höschenwindel;
- Fig. 2: einen Längsschnitt der Höschenwindel entlang der Linie 2-2 der Fig. 1;
- Fig. 3: einen Querschnitt der Höschenwindel entlang der Linie 3-3 der Fig. 2;
- Fig. 4: stark schematisiert eine mögliche Einrichtung zur Durchführung des Verfahrens.

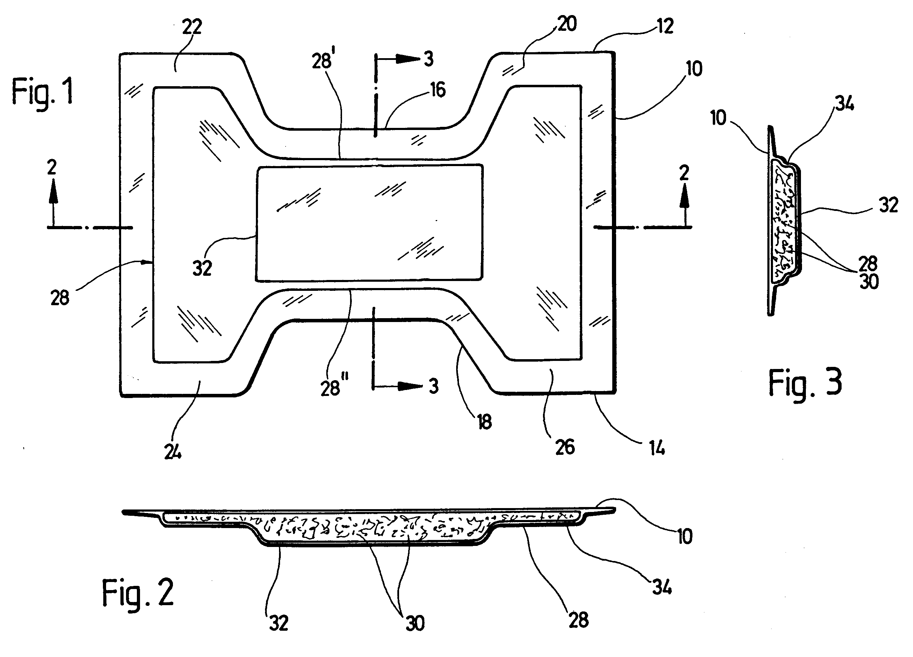

Die dargestellte Höschenwindel ist an ihrer einen, in Fig.1 unteren Seite mit einer Wäscheschutzfolie 10 ausgestattet, die vorzugsweise aus Polyäthylen besteht. Diese bildet beispielsweise einen im wesentlichen rechteckförmigen Zuschnitt, der im mittleren Bereich seiner beiden Längskanten 12 und 14 Beinausschnitte 16, 18 aufweist. Die beiden seitlich dieser Beinausschnitte 16, 18 liegenden Folienabschnitte 20, 22 bzw. 24, 26 umschliessen im angelegten Zustand der Höschenwindel den Körper im Bereich seiner Taille.

Mit 28 ist als Ganzes ein Saugkörper bezeichnet, der den Umrissen des Folienzuschnitts in kleineren Abmessungen in etwa entspricht. Dieser besteht aus einem Zellulosefasergemisch 30. Im Schrittbereich ist der Saugkörper 28 mit einer dicker auftragenden, vermehrt flüssigkeitsabsorbierenden Verstärkung 32 versehen, die im wesentlichen rechteckförmig ist und im Schrittbereich vorzugsweise vom Saugkörperrand 28′, 28˝ mit geringem Abstand endet und mit dem Saugkörpermaterial homogen ist.

Der Saugkörper 28 einschliesslich seiner Verstärkung 32 sind mit Quellstoffsubstanzen vermischt, deren Konzentration, ausgehend von der Verstärkung 32 in Richtung der Windellängsachse zu den Enden des Saugkörpers 28 hin abnimmt. Im Schrittbereich des Saugkörpers 28 beträgt hierbei deren Anteil bei einem bevorzugten Ausführungsbeispiel ungefähr 10 bis 15% Gewichtsprozent der gesamten Masse und in dessen Endbereichen 2 bis 5%. Das Gewicht der Zellstoffasern 32 pro Flächeneinheit ist im Schrittbereich mindestens 20% höher als im Endbereich, d.h. im Taillenbereich der Höschenwindel, woraus im Verstärkungsbereich eine erhöhte Saugkapazität für Körperflüssigkeiten resultiert.

Saugkörper 28 samt Verstärkung 32 nebst Wäscheschutzfolie 10 sind durch eine poröse Vliesstoffolie 34 ganzflächig abgedeckt, die beim Tragen zur Körperseite hin angelegt wird. Geht man beispielsweise für den Verstärkungsbereich 32 des Saugkörpers 28 von einer Konzentration an Quellstoffsubstanzen von 12% und einem Saugkörpergewicht pro Flächeneinheit von 600 g/m² sowie von einer Konzentration an Quellstoffsubstanzen für den übrigen Bereich des Saugkörpers 28 von 3% und einem Flächengewicht von 300 g/m² aus, so beträgt das Mengenverhältnis an Quellstoffsubstanzen im Fasermaterial im Schrittbereich das achtfache der Menge gegenüber seinem Umgebungsbereich. Es ist ersichtlich, dass man dadurch eine optimale Saugkapazität der Windel in demjenigen Bereich erhält, wo der stärkste Flüssigkeitsanfall stattfindet.

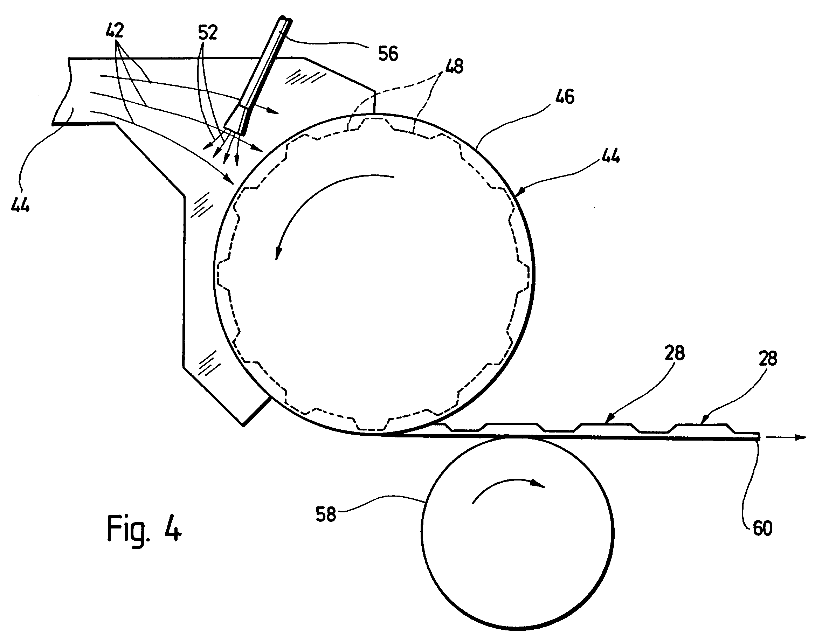

Die in Fig. 4 gezeigte Einrichtung weist einen Schacht 40 zur Führung eines ersten Luftstromes 42 auf. Dieser Schacht 40 nimmt an seinem einen Ende eine antreibbare Siebtrommel 44 auf und umschliesst diese teilweise. Sie weist an ihren Stirnenden überstehende Randteile 46 auf. Die Siebtrommel 44 ist am Trommelumfang mit Vertiefungen 48 ausgestattet, die durch breite Stege 50 voneinander getrennt sind. Vertiefungen 48 und Stege 50 nehmen durch den ersten Luftstrom 42 und einen zweiten Luftstrom 52 zugeführte, durchmischte Substanzen auf, und formen dabei einzelne, oder aber endlos aneinandergereihte Saugkörper 54.

Der zweite Luftstrom 52 wird über ein Rohr 56 ungefähr senkrecht zur Strömungsrichtung des ersten Luftstromes 42 und in diesen hineingeleitet,aus welchem Quellstoffsubstanzen austreten. Die vermischten Luftströme 42 und 52 werden durch eine an das Innere der Siebtrommel 44 angeschlossene Unterdruckquelle vom Trommelumfang abgesaugt, wobei sich das mitgeführte Substanzgemisch verdichtend am Trommelumfang anlagert.

Die sich hierbei bildende Materialbahn wird von der Siebtrommel 44 tangential abgezogen und über einer Walze 58 mittels eines Förderbandes 60 der weiteren Verarbeitung, wie Anbringen der Wäscheschutzfolie 10 und Vliesstoffabdeckung 32, Aufbringen von elastischen Bändern und Verschlusselementen, ggf. nach einem Trennschnitt, zugeführt. Danach wird die so komplettierte Materialbahn durch Quertrennen in einzelne Zellstoffartikel aufgeteilt.

Mit Hilfe des zweiten Luftstromes 52 werden die Quellstoffsubstanzen in pulsierend zunehmender und abnehmender Mengenkonzentration dem ersten Luftstrom 42 derart zugeführt, dass in den Verstärkungsbereichen der Saugkörper die maximale Saugfähigkeit erreicht wird. Die Mengenmaxima des pulsierenden, mit Quellstoffen beladenen Luftstromes werden jeweils dem Verstärkungsbereich 32 zugeordnet.

## Patentansprüche

1. Hygienischer Zellstoffartikel als Einmalartikel, wie Windel oder Höschenwindel, mit folgenden Merkmalen:
- einer Wäscheschutzfolie,
- einer porösen Vliesstoffabdeckung auf der Körperseite,
- einem zwischen Wäscheschutzfolie und Vliesstoffabdeckung befindlichen Saugkörper, der sich vom Schrittbereich bis in die Taillenbereiche erstreckt und aus einer Mischung von Zellulosefasern und Quellstoffsubstanzen besteht, wobei dieser Saugkörper einen sich in der Längsmitte der Windel erstreckenden, im Schrittbereich angeordneten und mit dem Saugkörper homogenen Verstärkungsbereich aufweist und die Quellstoffsubstanzen im Schrittbereich in grösserer Menge vorliegen als im Taillenbereich,
**dadurch gekennzeichnet,**
dass die Quellstoffsubstanzen in Richtung der Windellängsachse vom Verstärkungsbereich (32) nach aussen abnehmend entsprechend dem Verteilungsprofil der anfallenden Flüssigkeit derart aufgetragen sind, dass die Konzentration der Substanzen im Saugkörper im Verstärkungsbereich 8 % bis 40 % und in den Taillenbereichen 1 % bis 7 % des Gewichtes des Saugkörpers beträgt.

2. Zellstoffartikel nach Anspruch 1, dadurch gekennzeichnet, dass die Konzentration der Quellstoffsubstanzen im Schrittbereich des Saugkörpers ca. 10 % bis 15 % und im Taillenbereich 2 % bis 5 % beträgt.

3. Zellstoffartikel nach Anspruch 1 oder 2, dadurch gekennzeichnet, dass das Gewicht pro Flächeneinheit der Zellstoff-Fasern (30) im Schrittbereich mindestens 20 % höher ist als im Taillenbereich.

4. Verfahren zur Herstellung eines hygienischen Zellstoffartikels nach einem der vorhergehenden Ansprüche, gekennzeichnet durch folgende Verfahrensschritte:
a)
- permanentes Zuführen eines Zellstoffasergemisches mittels eines ersten Luftstromes (42) auf einen luftdurchlässigen, Vertiefungen (48) aufweisenden Träger (44);
- Zuführen von Quellstoffsubstanzen in pulsierend zunehmender und abnehmender Mengenkonzentration mittels eines zweiten Luftstromes (52) in Richtung auf den ersten Luftstrom (42) bei gleichzeitiger Durchmischung beider mitgeführter Substanzen, derart, dass das Mengenmaximum in die vorgesehenen Verstärkungsbereiche (32) der Saugkörper (28) gelangt;
- Absaugen des auf den durchlässigen Träger (44) auftreffenden Luftstromgemisches (42, 52) bei gleichzeitiger verdichtender Anlagerung beider durchmischter Substanzen auf diesem zu einer Materialbahn, derart, dass sich Saugkörper (28) und Verstärkungen (32) ausbilden;
b) Vereinigen der Saugkörper an Ober- und Unterseite mit Wäscheschutzfolie (10) und Vliesstoffabdeckung (32);
c) Querschneiden des komplettierten Bahnmaterials in Fertigprodukte.

5. Verfahren nach Anspruch 4, dadurch gekennzeichnet, dass ein dritter, gleichfalls Zellstoffasergemisch führender Luftstrom dem ersten Luftstrom (42) zur Bildung der Verstärkungen (32) der Saugkörper (28) intermittierend zugeführt wird.

## Claims

1. Disposable absorbent article, such as a diaper or diaper pants, having
- an underwear-protection foil;
- a porous fleece cover on the body side;
- an absorbent body arranged between the underwear-protection foil and the fleece cover, which body extends from the crotch region to the waist region and consists of a mixture of cellulose fibres and swelling substances, the absorbent body comprising a thicker area that is homogenous with the suction body and extends along the longitudinal centre of the diaper in the latter's crotch area, and the swelling substances being present in greater quantities in the crotch area than in the waist area,
**characterized in that**
the swelling substances are applied in quantities decreasing in the direction of the longitudinal axis of the diaper, from the thicker area (32) toward the outside, in conformity with the distribution profile of the liquid to be absorbed, and that the concentration of the substances in the absorbent body is between 8 % and 40 % in the thicker area and between 1 % and 7 % in the waist area, related to the weight of the absorbent body.

2. Absorbent article according to claim 1, characterized in that the concentration of the swelling substances is between approximately 10 % and 15 % in the crotch area of the absorbent body, and between 2 % and 5 % in the waist area.

3. Absorbent article according to claim 1 or 2, characterized in that the weight of the cellulose fibres (30) per surface area unit is at least 20 % higher in the crotch area than in the waist area.

4. Process of production of a disposable absorbent article according to any of the preceding claims, characterized by the steps of
a)
- feeding permanently, by means of a first air jet (42), a cellulose fibre mixture onto an air-permeable carrier (44) provided with deepened portions (48);
- feeding swelling substances by means of a second air jet (52) toward the first air jet (42), in volume concentrations which increase and decrease in a pulsating way, while simultaneously mixing the two substances carried by the air jets in a manner such that a maximum of the quantities is deposited in the planned thicker areas (32) of the absorbent bodies (28),
- evacuating the mixed air jets (42, 52) impinging upon the permeable carrier (44), whereby the two mixed substances are simultaneously compacted and deposited on the carrier in the form of a material web so as to form the absorbent body (28) and the thicker portions (32);
b)
- uniting the upper and lower faces of the absorbent body with the underwear-protection foil (10) and the fleece cover (32), respectively; and
c)
- cross-cutting the completed web material into finished products.

5. Method according to claim 4, characterized in that a third air jet, likewise carrying the cellulose fibre mixture, is intermittently introduced into the first air jet (42) for forming the thicker portions (32) of the absorbent bodies (28).

## Revendications

1. Article d'hygiène en matière cellulosique, à usage unique, tel qu'une couche ou une couche culotte, présentant les caractéristiques suivantes :
- un film protecteur de la lingerie,
- un recouvrement poreux en matière non tissée du côté du corps,
- un corps absorbant placé entre le film protecteur de la lingerie et le recouvrement en matière non tissée, qui s'étend de l'entre-jambe aux zones de la taille et qui est constitué d'un mélange de fibres cellulosiques ou de substances à base de matières hydro-absorbantes, tandis que ledit corps absorbant présente une zone renforcée s'étendant au centre de la couche, disposée dans l'entre-jambe et homogène avec le corps absorbant, les substances à base de matières hydro-absorbantes étant présentes en plus grandes quantités dans l'entre-jambe que dans la zone de la taille, caractérisé en ce que, les substances à base de matières hydro-absorbantes sont appliquées de manière à diminuer progressivement vers l'extérieur à partir de la zone de renfort (32) et en suivant l'axe longitudinal de la couche, compte-tenu du profil de distribution de l'apport du liquide, de telle sorte que la concentration des substances dans le corps absorbant soit, dans la zone de renfort, comprise entre 8 et 40 % et dans les zones de la taille entre 1 et 7 % en poids dudit corps absorbant.

2. Article en matière cellulosique selon la revendication 1, caractérisé en ce que la concentration des substances à base de matières hydro-absorbantes du corps absorbant est comprise dans l'entre-jambe entre environ 10 et 15 % et dans la zone de la taille entre 2 et 5 %.

3. Article en matière cellulosique selon la revendication 1 ou 2, caractérisé en ce que le poids par unité de surface des fibres cellulosiques (30) est, dans l'entre-jambe, supérieur d'au moins 20 % à celui de la zone de la taille.

4. Procédé pour la fabrication d'un article d'hygiène en matière cellulosique, selon l'une des revendications précédentes, caractérisé en ce qu'il comprend les étapes opérationnelles suivantes consistant à :
a) réaliser un apport permanent d'un mélange de fibres cellulosiques au moyen d'un premier flux d'air (42) sur un support (44) présentant des empreintes (48) perméables à l'air ;
réaliser un apport de substances à base de matières hydro-absorbantes en une concentration quantitative augmentant et diminuant de manière pulsée, au moyen d'un deuxième flux d'air (52) dirigé sur le premier flux d'air (42), avec mélange simultané des deux substances apportées, de telle sorte que le maximum quantitatif parvienne dans les zones de renfort prévues (32) du corps absorbant (28) ;
- aspirer le mélange apporté par les flux d'air (42, 52) sur le support perméable (44), avec simultanément application et compactage des deux substances intimement mélangées sur ledit Support (44) afin de former une nappe de tissu, de manière à former des corps absorbants (28) et des renforts (32) ;
b) réunir les corps absorbants, sur le côté inférieur et le côté supérieur, et un film protecteur de la lingerie (10) et un recouvrement en matière non tissée (32) ;
c) découper en direction transversale de la nappe complète de tissu en produits finis.

5. Procédé selon la revendication 4, caractérisé par l'apport intermittent d'un troisième flux d'air conduisant également un mélange de fibres cellulosiques vers le premier flux d'air (42), afin de former les renforts (32) du corps absorbant (28).
